# EUROPEAN PATENT APPLICATION

(11) **EP 1 837 343 A1**
(43) Date of publication of application: **26.09.2007**
(21) Application number: 05814172.2
(22) Date of filing: 07.12.2005
(51) Int. Cl.: C07J 21/00

(54) **PROCESS FOR PRODUCING (5 ALPHA, 7 ALPHA)-3-SPIRO-2'-(1',3'-DIOXOLAN)-24-OXOCHOLEST-22-EN-7-YL BENZOATE**

(30) Priority: 08.12.2004 JP 2004354787
(71) Applicant: KURARAY CO., LTD., Kurashiki-shi, Okayama 710-8622 (JP)
(72) Inventor: KOYAKUMARU, Kenichi, c/o KURARAY CHEMICAL CO., LTD, Bizen-shi, Okayama 7050025 (JP); UJITA, Katsuji, c/o KURARAY CO., LTD, Tainai-shi, Niigata 9592691 (JP)
(74) Representative: Duckworth, Timothy John
(86) International application number: PCT/JP2005/022457
(87) International publication number: WO 2006/062129

(57) **Abstract**

The present invention provides a method of producing (5α,7α)-3-spiro-2'-(1',3'-dioxolan)-24-oxocholest-22-en-7-yl benzoate represented by the formula (I) 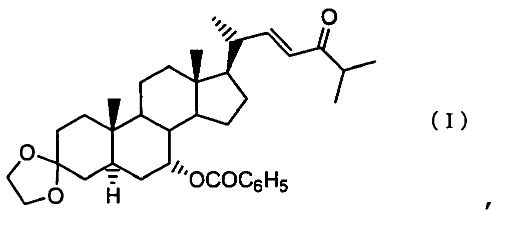 including dissolving a crude product containing (5α,7α)-3-spiro-2'-(1',3'-dioxolan)-24-oxocholest-22-en-7-yl benzoate, which is obtained by a reaction step, in methanol within the range of 1.5 - 3 in a mass ratio relative to the contained (5α,7α)-3-spiro-2'-(1',3'-dioxolan)-24-oxocholest-22-en-7-yl benzoate to give a solution, adding the solution to water within the range of 5 - 30 in a mass ratio relative to the contained (5α,7α)-3-spiro-2'-(1',3'-dioxolan)-24-oxocholest-22-en-7-yl benzoate to solidify (5α,7α)-3-spiro-2'-(1',3'-dioxolan)-24-oxocholest-22-en-7-yl benzoate to give a suspension, separating the solid from the suspension, and drying the obtained solid.

The compound obtained by the present invention is useful as a production intermediate for squalamine having potent antibacterial activity and antitumor activity.

## Description

### Technical Field

The present invention relates to a production method of (5α,7α)-3-spiro-2'-(1',3'-dioxolan)-24-oxocholest-22-en-7-yl benzoate useful as a synthetic intermediate for squalamine.

### Background Art

Squalamine shown by the formula (V)

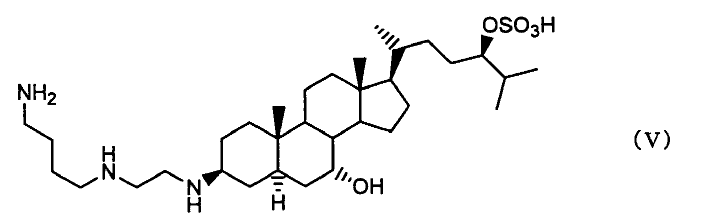

is a compound reported to have a strong antibacterial activity against Gram-positive bacteria, Gram-negative bacteria, fungi and the like, as well as antitumor activity, and drawing attention as a new antibiotic.

Conventionally, squalamine has been extracted from the shark liver. However, since the content ratio thereof is extremely low, as much as 0.001 - 0.002 mass %, and the extraction efficiency is poor, various studies of chemical synthesis methods have been conducted. Particularly, (5α,7α)-3-spiro-2'-(1',3'-dioxolan)-24-oxocholest-22-en-7-yl benzoate shown by the formula (I)

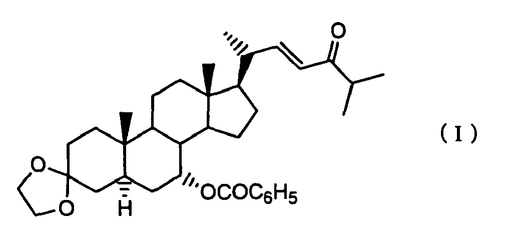

(hereinafter sometimes to be referred to as unsaturated ketone (I)) is known as an important synthetic intermediate for squalamine (patent reference 1, non-patent reference 1).

Conventionally, as a method for producing unsaturated ketone (I), a method comprising
step (a'): adding an aqueous potassium bromide solution to a solution of an alcohol compound of the following formula (IV)

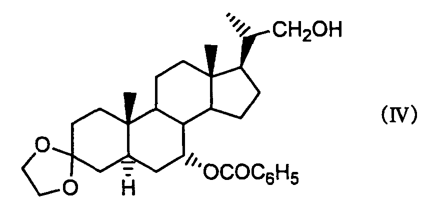

(hereinafter sometimes to be referred to as alcohol (IV)) and 2,2,6,6-tetramethylpiperidine N-oxyl in methylene chloride and adding an aqueous sodium hypochlorite solution adjusted to pH 9.5 by adding sodium hydrogen carbonate to give an aldehyde compound of the following formula (II)

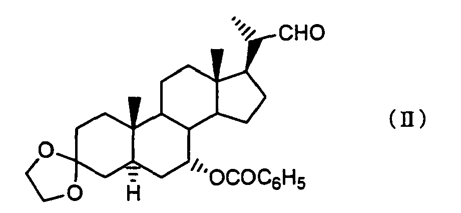

(hereinafter sometimes to be referred to as aldehyde (II)) (hereinafter sometimes to be referred to as step (a')); and step (b'): reacting aldehyde (II) obtained in the aforementioned step (a') with a reaction product of diethyl (3-methyl-2-oxobutyl)phosphonate of the following formula (III')

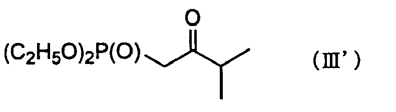

(hereinafter sometimes to be referred to as compound (III')) and sodium hydride to give unsaturated ketone (I) (hereinafter sometimes to be referred to as step (b'), is known (patent reference 1, non-patent reference 1).

patent reference 1: WO 98/24800
non-patent reference 1: Journal of Organic Chemistry, 1998, vol. 63, p. 3786

### Disclosure of the Invention

### Problems to be Solved by the Invention

However, unsaturated ketone (I) is a compound generally showing gummy property, which makes purification by recrystallization difficult, which is one of the industrially advantageous purification methods. According to the above-mentioned method including step (a') and step (b') (patent reference 1, non-patent reference 1), 499 mg of unsaturated ketone (I) is obtained as a solid having a melting point of 85 - 121°C in a yield of 90% by a method comprising dissolving a crude product in methanol (5 ml) containing 4 drops of pyridine, dropwise adding the obtained solution to 100 ml of water with shaking to allow solidification, collecting the obtained solid by filtration and washing 3 times with 20 ml of water.

By this method, it is necessary to use a large amount of methanol as a solvent and water which is a poor solvent necessary for precipitating unsaturated ketone (I) as a solid for the amount of the obtained unsaturated ketone (I), and the range of the melting point of the obtained unsaturated ketone (I) is wide and the purity is not entirely high. Therefore, there is a large room for improvement for industrial application, from the aspects of producibility and production cost.

Accordingly, the problem to be solved by the present invention is provision of a method capable of producing unsaturated ketone (I), which can be used as a synthetic intermediate for squalamine, at high purity and high recovery yield with good producibility, ease of operation and industrial advantages without using a large amount of a solvent and a large amount of a poor solvent necessary for precipitating unsaturated ketone (I) as a solid.

### Means of Solving the Problems

Unsaturated ketone (I) is insoluble in water. The present inventors took note that the method disclosed in patent reference 1 and non-patent reference 1 for obtaining unsaturated ketone (I) as a solid is the same as the isolation operation which is what is called a reprecipitation operation in the field of polymer chemistry, and made a hypothesis that unsaturated ketone (I) can be precipitated as a solid in a mixture of water and a water-miscible solvent by dissolving unsaturated ketone (I) in a water-miscible solvent such as methanol that is miscible with water and can dissolve unsaturated ketone (I), and then adding the solution to water, and made close studies on the conditions such as the kind and the amount of water-miscible solvent to be used for dissolving unsaturated ketone (I), the amount of water to be used, temperature and the like. As a result, we have found that a precipitation operation of unsaturated ketone (I) using methanol and water within a particular mass ratio range relative to unsaturated ketone (I) unexpectedly affords unsaturated ketone (I), which has a narrower melting temperature range than the prior art and high purity, and which can be used as a synthetic intermediate for squalamine, conveniently at a high recovery yield without using a large amount of a solvent and a large amount of a poor solvent necessary for precipitating unsaturated ketone (I) as a solid, which resulted in the completion of the present invention.

Accordingly, the present invention is shown as follows.

[1] A method of producing unsaturated ketone (I) comprising dissolving a crude product containing unsaturated ketone (I), which is obtained by a reaction step, in methanol within the range of 1.5 - 3 in a mass ratio relative to the contained unsaturated ketone (I) to give a solution, adding the solution to water within the range of 5 - 30 in a mass ratio relative to unsaturated ketone (I) to solidify unsaturated ketone (I) to give a suspension, separating the solid from the suspension, and drying the obtained solid.
[2] The production method of [1], wherein the reaction step is a step for condensing aldehyde (II) and a ketophosphonate metal salt of the formula (III)

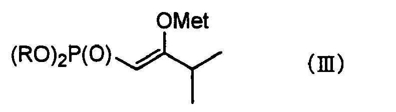

wherein R is an alkyl group or an aryl group, and Met is a metal atom (hereinafter to be referred to as metal phosphonate (III)).
[3] The production method of [2], wherein the aldehyde (II) is obtained by oxidizing a mixture of a solution containing alcohol (IV) and a buffer with hypochlorite added as an oxidant, in the presence of an N-oxyl compound and within the pH range of an aqueous layer of 7 - 11.

### Effect of the Invention

According to the present invention, unsaturated ketone (I), useful as a synthetic intermediate for squalamine having antibacterial activity and antitumor activity, can be produced at high purity and high recovery yield with good producibility, ease of operation and industrial advantages.

### Best Mode for Embodying the Invention

Unsaturated ketone (I) can be preferably produced by condensation of aldehyde (II) and metal phosphonate (III).

The metal phosphonate (III) to be used for this reaction step can be obtained by reacting the corresponding ketophosphonate of the following formula (III")

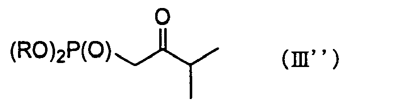

wherein R is as defined above) (hereinafter to be referred to as phosphonate (III")) with a basic substance containing a metal.

In the formula (III) and the formula (III"), the alkyl group for R preferably has 1 to 4 carbon atoms, for example, methyl group, ethyl group, propyl group, isopropyl group, butyl group and the like can be mentioned. The aryl group for R preferably has 6 to 10 carbon atoms, for example, phenyl group, tolyl group, naphthyl group and the like can be mentioned. As the metal atom for Met, alkali metal atoms such as lithium, sodium, potassium and the like can be mentioned.

As a basic substance to be reacted with phosphonate (III") to produce metal phosphonate (III), those generally used for producing the corresponding metal salt from ketophosphonate in the field of organic synthesis chemistry can be used. For example, metal hydrides such as sodium hydride, potassium hydride and the like; alkali metal alkoxides such as sodium methoxide, sodium ethoxide, sodium t-butoxide, sodium t-amyloxide, potassium t-butoxide and the like; alkyl metals such as methyllithium, n-butyllithium and the like; metal amides such as sodium amide, potassium amide, lithiumdiisopropylamide, lithium diethylamide, lithium hexamethyldisilazide, sodium hexamethyldisilazide, potassium hexamethyldisilazide and the like, and the like can be used. From the aspects of easiness of industrial handling, easiness of controlling the reaction and the like, alkali metal alkoxides (e.g., metal alkoxide) of tertiary alcohol, particularly sodium t-butoxide and the like, are preferable.

The amount of the basic substance to be used is generally within the range of 0.8 to 2 mol per 1 mol of phosphonate (III"). When an excess basic substance remains, however, it may cause an adverse influence such as a concurrent side reaction of epimerization of the methyl group at the 20-position of aldehyde (II) and the like during the reaction with aldehyde (II), and the like. Preferably, therefore, the amount of the basic substance is, as far as possible, within the range that does not cause basic substance to remain. Specifically, it is preferably within the range of 0.8 to 1.1 mol per 1 mol of phosphonate (III").

The reaction of phosphonate (III") with a basic substance is generally carried out in a solvent. As the solvent to be used, for example, ethers such as tetrahydrofuran, dioxane, dimethoxyethane and the like; aromatic hydrocarbons such as toluene and the like; dimethyl sulfoxide, N,N-dimethylformamide, N-methylpyrrolidone and the like can be mentioned. While the amount of the solvent to be used is not particularly limited, it is generally within the range of 2-to 50-fold by mass relative to phosphonate (III"). The reaction temperature is generally within the range of -20°C to 50°C.

The thus-obtained metal phosphonate (III) is generally used for the reaction with aldehyde (II) without purification. The reaction is generally carried out in a solvent. Usable solvents are those similar to the solvents used for the reaction of phosphonate (III") with a basic substance. From the industrial viewpoints, it is advantageous that the solvent used for the reaction of phosphonate (III") with a basic substance is the same as that used for the reaction of the obtained metal phosphonate (III) with aldehyde (II), and ethers such as tetrahydrofuran and the like are particularly preferable.

This reaction is generally carried out according to a method including adding metal phosphonate (III) or a solution thereof to a solution obtained by dissolving aldehyde (II) in a solvent, or adding a solution of aldehyde (II) to metal phosphonate (III) or a solution thereof. The amount of aldehyde (II) to be used is generally within the range of 0.5 to 2 mol per 1 mol of phosphonate (III"). When aldehyde (II) remains after the reaction, separation from the object resultant product, unsaturated ketone (I), becomes difficult. Therefore, it is preferable to consume aldehyde (II) as much as possible in the reaction. More specifically, the amount of aldehyde (II) to be used is preferably within the range of 0.5 to 0.95 mol per 1 mol of phosphonate (III").

The amount of the solvent to be used for dissolving aldehyde (II) is generally within the range of 2- to 50-fold by mass relative to aldehyde (II). The reaction temperature is generally within the range of -20°C to 50°C. While the reaction time may vary depending on the kind or the ratio of the amount of metal phosphonate (III) used, reaction temperature and the like, it is generally within the range of 0.5 - 48 hr.

A crude product containing unsaturated ketone (I) can be obtained from the resulting reaction mixture by an isolation operation employed in conventional organic synthesis chemistry. For example, water or an aqueous sodium hydrogen carbonate solution is added to the reaction mixture, the organic layer is diluted with an organic solvent as necessary, the organic layer and the aqueous layer are separated, the aqueous layer is extracted with an organic solvent (e.g., isopropyl ether, ethyl acetate and the like), the extract is combined with the organic layer previously separated, the organic layer is washed with alkali aqueous solution as necessary, and concentrated to give a crude product containing unsaturated ketone (I).

By dissolving the thus-obtained crude product containing unsaturated ketone (I) in methanol within the range of 1.5 to 3 mass ratio relative to unsaturated ketone (I) contained in the crude product, and adding the obtained methanol solution to water within the range of 5 to 30 mass ratio relative to unsaturated ketone (I) contained in the crude product by a method such as dropwise addition and the like, a suspension of unsaturated ketone (I) dispersed as an industrially handlable ultrafine solid can be obtained. The amount ratio of methanol and water to be used is important. When, during this operation, the amount of methanol to be used relative to unsaturated ketone (I) contained in a crude product is lower than 1.5 in a mass ratio, the crude product of unsaturated ketone (I) is not completely dissolved, and when it exceeds 3, the amount of water necessary for increasing the recovery rate of unsaturated ketone (I) becomes larger, which in turn necessitates use of a large amount of a solvent and a large amount of a poor solvent necessary for precipitation of unsaturated ketone (I) as a solid. In addition, when the amount of water relative to unsaturated ketone (I) contained in the crude product is lower than 5 in the mass ratio, the recovery rate of unsaturated ketone (I) decreases and, in an extreme case, the whole system is solidified and it becomes extremely difficult to take out unsaturated ketone (I), as in the below-mentioned Comparative Examples. On the other hand, when it exceeds 30, the amount of the solvent and the poor solvent necessary for precipitating the resultant product as a solid increases, which is disadvantageous from the aspect of the production cost.

Even if the amounts of the solvent and water as a poor solvent are defined to fall within the range of the present invention, when a solvent other than methanol, for example, ethanol, tetrahydrofuran, acetonitrile and the like is used as a water-miscible solvent to dissolve a crude product containing unsaturated ketone (I), dropwise addition of a solution dissolving the crude product to water does not result in the solidification of unsaturated ketone (I) but results in separation of an oil, thus failing to obtain unsaturated ketone (I) as a solid. Alternatively, when a solution dissolving the crude product is added dropwise to water, a dispersed suspension is not obtained but gummy solidification occurs, thus making it extremely difficult to take out the product. Accordingly, a suspension wherein a fine solid permitting industrial handling is precipitated and dispersed, like the one obtained using methanol, cannot be produced. Therefore, industrial handling becomes difficult or impossible.

During addition of a solution of a crude product containing unsaturated ketone (I) in methanol to water, water may be stirred or shaken to afford a suspension permitting easy industrial handling. While the temperature during addition can be appropriately selected, generally, it is preferably within the range of 5°C - 30°C. While the time of addition can be appropriately selected, since too fast an addition may provide a suspension industrially difficult to handle, and addition over too long a time degrades producibility, generally, it is preferably within the range of 5 min - 2 hr (for example, dropwise addition and the like). Moreover, it is possible to age the suspension after addition. For aging, the suspension is generally stirred at a temperature within the range of -10°C to 30°C for 5 min - 3 hr.

Industrially available methanol may contain an acidic substance such as formic acid and the like. To avoid adverse influences due to the acidic substance such as elimination of an acetal-protecting group and the like of unsaturated ketone (I), isomerization of double bond, epimerization of methyl group at the 20-position and the like, methanol used for dissolving a crude product containing unsaturated ketone (I) may contain a basic substance. As the basic substance, for example, pyridine, sodium hydrogen carbonate and the like can be used. The amount thereof to be used is generally within the range of 1- to 3-fold relative to the amount necessary for neutralizing the acidic substance contained in methanol.

For the same reason, water to which a methanol solution of a crude product containing unsaturated ketone (I) is added may contain a basic substance such as sodium hydrogen carbonate and the like. The amount thereof to be used is generally within the range of 1- to 3-fold relative to the amount necessary for neutralizing the acidic substance contained in methanol.

Unsaturated ketone (I) can be obtained by separating a solid from the thus-obtained suspension. As a method for separating a solid, for example, a method generally employed industrially can be appropriately selected, such as filtration under reduced pressure, pressurization filtration, centrifugal filtration and the like. In this case, for smooth separation of the solid, the separation operation is preferably performed at a temperature when the suspension was added, or when the suspension was aged, at a temperature of aging.

Unsaturated ketone (I) can be produced with a high purity and a high recovery yield by washing the obtained solid with water and drying the solid as necessary.

On the other hand, aldehyde (II) can be obtained by oxidizing the hydroxyl group of alcohol (IV). While the oxidation can be performed using various oxidants, for example, a method including oxidizing with a hypochlorite such as sodium hypochlorite and the like, in the presence of an N-oxyl compound catalyst such as 2,2,6,6-tetramethylpiperidine N-oxyl, 4-methoxy-2,2,6,6-tetramethylpiperidine N-oxyl, 4-acetoxy-2,2,6,6-tetramethylpiperidine N-oxyl and the like, and the like can be used. This method is described in detail in the following.

The oxidation reaction is generally carried out in a solvent. The solvents to be used include, for example, halogenated hydrocarbons such as dichloromethane, 1,2-dichloroethane, chloroform and the like; esters such as ethyl acetate, butyl acetate and the like; aromatic hydrocarbons such as toluene, xylene, mesitylene and the like; hydrocarbons such as pentane, hexane, heptane, octane and the like; and the like. These solvents are not miscible with water. Since hypochlorite to be added as an oxidant is in the form of an aqueous solution generally industrially available, the reaction system generally includes two phases. While the amount of the solvent to be used is not particularly limited, it is generally within the range of 5- to 50-fold by mass relative to alcohol (IV).

The amount of hypochlorite to be used is generally within the range of 0.8- to 2-fold relative to the amount of the oxidant necessary for oxidizing the hydroxyl group of alcohol (IV). When its amount of use is less, alcohol (IV), the starting material, may remain to render purification difficult, and when its amount of use is in excess, aldehyde (II), the resultant product, may be further oxidized to possibly lower the yield. Therefore, it is preferable to use, as far as practical, hypochlorite only in an amount necessary for consuming alcohol (IV). Specifically, a method including tracing the conversion rate of alcohol (IV) during the reaction and terminating addition of hypochlorite when disappearance of alcohol (IV) is confirmed, and the like can be employed.

Hypochlorite is preferably added as an aqueous solution of pH 11 - 12, from the viewpoint of its stability. However, when the pH of the aqueous phase of the reaction system becomes too high due to the addition, a side reaction such as epimerization of the methyl group at the 20-position of the resultant product (aldehyde (II)) and the like is highly likely to occur concurrently. Therefore, the reaction is preferably carried out while controlling the pH of the aqueous phase. The controlled pH range of the aqueous phase is preferably 7 - 11, in consideration of the rate of oxidation reaction, possibility of concurrent side reaction and the like. As a method of controlling pH, for example, a method including previously adding a buffer capable of adjusting the aqueous phase to pH 7 - 11 to the aqueous phase, a method including previously adding a substance having a buffering ability, a method including adding an acidic substance along with the addition of an aqueous hypochlorite solution and the like can be employed. As a substance having a buffering ability, sodium hydrogen carbonate and the like can be used.

While the amount of an N-oxyl compound to be used as a catalyst is not particularly limited, it is generally within the range of 0.001 - 0.10 mol, more preferably within the range of 0.001 - 0.05 mol, relative to alcohol (IV).

The reaction temperature is preferably within the range of -20°C to 70°C, more preferably within the range of -10°C to 40°C. The reaction time varies depending on the reaction conditions, and can change according to the conversion rate of alcohol (IV) actually traced. Industrially, it is generally within the range of 0.5 - 10 hr.

In this oxidation reaction, a promoter can be used for a smooth reaction. As the promoter, for example, lithium bromide, sodium bromide, potassium bromide, lithium iodide, sodium iodide, potassium iodide and the like can be used. Of these, sodium bromide and potassium bromide are preferable. When a promoter is further used, the amount thereof to be added is generally within the range of 0.05 - 0.5 mol per 1 mol of alcohol (IV).

The method for isolation and purification of aldehyde (II) obtained by such oxidation reaction is not particularly limited, and a method generally used for isolation and purification of an organic compound, which includes recrystallization or column chromatography and the like can be employed after extraction operation and the like. In addition, it is also possible to concentrate an extract without purification, dissolve the extract in a solvent used for the reaction in the next step and use the same for the next step.

### Examples

The present invention is specifically explained in the following by referring to Examples, which are not to be construed as limitative.

### Reference Example 1: production of (5α,7α,20S)-3-spiro-2'-(1',3'-dioxolan)-20-methyl-21-oxopregnane-7-yl benzoate (aldehyde (II))

Under a nitrogen atmosphere, a mixture of (5α,7α,20S)-3-spiro-2'-(1',3'-dioxolan)-20-methyl-21-hydroxypregnane-7,21-diol 7-benzoate(alcohol(I)) (content 104 g, 209 mmol), ethyl acetate (569 g), 2,2,6,6-tetramethylpiperidine N-oxyl (167 mg, 1 mmol), potassium bromide (5.1 g, 42 mmol), sodium hydrogen carbonate (5.3 g, 63 mmol) and water (54 g) in two layers was stirred in a 1000 ml flask at an inside temperature of 0°C - 5°C. A 12 mass % aqueous sodium hypochlorite solution (138 g, 239 mmol) was added dropwise, in 4 portions of 49 g, 48 g, 37 g and 4 g, to the mixture while maintaining the inside temperature at 0°C - 5°C and, after the completion of each addition, the conversion rate of alcohol (I) was analyzed by high performance liquid chromatography (HPLC). During this period, the pH of the reaction mixture was maintained within the range of 8 - 10.
After confirmation of the disappearance of alcohol (I), sodium sulfite (42 mg, 0.3 mmol) was added to the reaction mixture at 0 - 2°C and the mixture was stirred to decompose excess sodium hypochlorite remaining in the reaction mixture. The organic layer was separated, washed twice with degassed 3 mass % brine (285 g + 40 g) and confirmed that the washed aqueous layer had a pH within the range of 7 - 8. The organic layer was concentrated at 50°C, and the solvent was substituted with tetrahydrofuran to make tetrahydrofuran and ethyl acetate ratio become 98 to 2, to give a tetrahydrofuran solution (440 g) containing aldehyde (II) (content 103 g, 208 mmol, yield 99%).

### Example 1: (5α,7α)-3-spiro-2'-(1',3'-dioxolan)-24-oxocholest-22-en-7-yl benzoate (production of unsaturated ketone (I))

Under a nitrogen atmosphere, a suspension of sodium t-butoxide (22 g, 229 mmol) in tetrahydrofuran (278 g) was stirred in a 1000 ml flask, and the mixture was to give a suspension. To this suspension was added dropwise diethyl (3-methyl-2-oxobutyl)phosphonate (57 g, 250 mmol) at an inside temperature of 20 - 25°C over 1 hr and the mixture was stirred for 10 min. Then, to this solution was added dropwise a solution (439 g; aldehyde (II) content 103 g, 208 mmol) of (5α,7α,20S)-3-spiro-2'-(1',3'-dioxolan)-20-methyl-21-oxopregnan-7-yl benzoate (aldehyde (II)) obtained in Reference Example 1 in tetrahydrofuran at an inside temperature of 20 - 25°C over 1 hr, and the mixture was reacted at the same temperature for 20 hr.
After confirmation that the residual amount of aldehyde (II) was less than 1% by means of HPLC, the reaction was quenched by the addition of 2 mass % aqueous sodium hydrogen carbonate solution (174 g) to the reaction mixture. Isopropyl ether (139 g) was further added and the organic layer and the aqueous layer were separated. The aqueous layer was extracted with isopropyl ether (139 g). The extract and the organic layer separated earlier were combined, and the mixture was washed with 5 mass % aqueous sodium carbonate (87 g x 4 times), removing 20-carboxylic acid byproduct, to make it less than 1% of the object compound (unsaturated ketone (I)). The organic layer was washed with 2 mass % aqueous sodium hydrogen carbonate solution (87 g) and the mixture was concentrated at 60°C. At this time point, it was confirmed by HPLC that unsaturated ketone (I) was contained in the concentrate (net. 113 g, 201 mmol). The obtained concentrate was dissolved in methanol containing 1.5 mass % of pyridine, thereby affording contained unsaturated ketone (I)/methanol=1/2 (mass ratio).
The obtained methanol solution was added dropwise to water (1128 g, 9.98-fold by mass relative to unsaturated ketone (I)), in which sodium hydrogen carbonate (1 g) had been dissolved, at 15°C - 20°C over 1 hr. After completion of the dropwise addition, the obtained suspension was stirred at 0 - 4°C for 1 hr, and the mixture was filtered at the same temperature. The obtained solid was washed with water, and dried at 50°C for 10 hr under reduced pressure to give unsaturated ketone (I) having the following property as an off-white powder (117 g, purity 95% (by HPLC analysis),
melting point: 87 - 92°C).

¹H-NMR spectrum (270MHz, CDCl₃, TMS, ppm) δ: 0.71(s,3H), 0.88(s,3H), 1.07-2.00(m,30H), 2.18-2.30(m,1H), 2.79(heptet,1H,J=6.9Hz), 3.87(brs,4H), 5.15(brs,1H), 6.03(d,1H,J=15.8Hz), 6.64(dd, 1H,J=8.9Hz,15.8Hz), 7.47(t,2H,J=6.9Hz), 7.58(t,1H,J=6.9Hz), 8.05(d,2H,J=6.9Hz)

### Examples 2 - 5

The concentrate obtained in the same manner as in Example 1 was dissolved in methanol containing 1.5 mass % of pyridine at an amount ratio shown in Table 1 (ratio of amount used (mass ratio) relative to unsaturated ketone (I)), and added dropwise to water to give unsaturated ketone (I) as an industrially handlable powder.

[Table 1]

**Table 1**

| | Ex. 2 | Ex. 3 | Ex. 4 | Ex. 5 |
|---|---|---|---|---|
| content (g) of unsaturated ketone (I) | 0.37 | 1.0 | 1.0 | 2.8 |
| amount of methanol used (g (mass ratio)) | 0.8(2.16) | 2(2) | 2(2) | 5.3(1.89) |
| amount of water used (g (mass ratio)) | 10 (27) | 7 (7) | 10 (10) | 28 (10) |
| temperature and time of aging | No aging | 20°C, 1 hr | 0°C, 1 hr | 0°C, 1 hr |
| obtained amount (g) | 0.37 | 0.96 | 0.97 | 2.5 |
| melting point (°C) | 85 - 95 | 86 - 93 | 86 - 92 | 86 - 92 |

| | | | | |
|---|---|---|---|---|
| * temperature during addition: 20°C | | | | |

In patent reference 1 and non-patent reference 1, to obtain 499 mg of unsaturated ketone (I), a crude product was dissolved in methanol (5 ml, 10-fold by mass relative to unsaturated ketone (I)) containing a small amount of pyridine, and the solution was added dropwise to 100 ml of water (200-fold by mass relative to unsaturated ketone (I)) to allow solidification. As a result, only unsaturated ketone (I) having a broad melting point of 85°C - 121°C and having a low purity could be obtained. In contrast, from the above-mentioned results of the Examples, it is appreciated that unsaturated ketone (I) having a narrower melting point range can be obtained at a high recovery rate by similar solidification using methanol and water in particular mass ranges relative to unsaturated ketone (I) contained in the crude product obtained by the reaction. The amounts of methanol and water in the particular mass ranges here are smaller than the amounts of methanol and water as disclosed in patent reference 1 or non-patent reference 1. It is an unexpected and satisfactory result even for the present inventors that a compound having a purity equivalent to or not less than that of conventionally-obtained compounds and usable as an intermediate for a pharmaceutical product, which is the original object, can be obtained by such an operation using a small amount of a solvent.

### Comparative Examples 1 - 4

The crude reaction products obtained in the same manner as in Example 1 were dissolved in various solvents, and added dropwise to water. The solvent species and amount of use thereof (amount ratio (mass ratio) relative to unsaturated ketone (I)), and the amount of water used (amount ratio (mass ratio) relative to unsaturated ketone (I)) are summarized and shown in Table 2 together with the results. In every case, unsaturated ketone (I) could not be obtained as an easily handlable solid.

[Table 2]

**Table 2**

| | content (g) of unsaturated ketone (I) | solvent species and amount of use (g (mass ratio)) | amount of water used (g (mass ratio)) | property of precipitate |
|---|---|---|---|---|
| Comp. Ex. 1 | 0.5 | methanol 0.8(1.6) | 1(2) | whole system was solidified |
| Comp. Ex. 2 | 1.0 | acetonitrile 2.0(2.0) | 10(10) | gummy |
| Comp. Ex. 3 | 1.0 | tetrahydrofuran 2.0(2.0) | 10(10) | gummy |
| Comp. Ex. 4 | 1.5 | ethanol 6.0(4.0) | 2(1.33) | oily |

| | | | | |
|---|---|---|---|---|
| * temperature during addition: 20°C | | | | |

### Industrial Applicability

Unsaturated ketone (I) useful as a production intermediate for squalamine having potent antibacterial activity and antitumor activity can be industrially advantageously produced without using a large amount of a solvent and a large amount of water which is a poor solvent necessary for solidifying a resultant product.

## Claims

1. A method of producing (5α,7α)-3-spiro-2'-(1',3'-dioxolan)-24-oxocholest-22-en-7-yl benzoate represented by the formula (I) 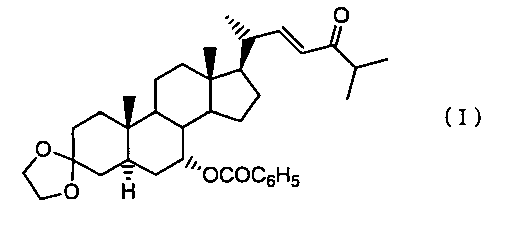 comprising dissolving a crude product containing (5a,7a)-3-spiro-2'-(1',3'-dioxolan)-24-oxocholest-22-en-7-yl benzoate, which is obtained by a reaction step, in methanol within the range of 1.5 - 3 in a mass ratio relative to the contained (5α,7α)-3-spiro-2'-(1',3'-dioxolan)-24-oxocholest-22-en-7-yl benzoate to give a solution, adding the solution to water within the range of 5 - 30 in a mass ratio relative to the contained (5α,7α)-3-spiro-2'-(1',3'-dioxolan)-24-oxocholest-22-en-7-yl benzoate to solidify (5α,7α)-3-spiro-2'-(1',3'-dioxolan)-24-oxocholest-22-en-7-yl benzoate to give a suspension, separating the solid from the suspension, and drying the obtained solid.

2. The production method of claim 1, wherein the reaction step is a step for condensing aldehyde compound represented by the formula (II) 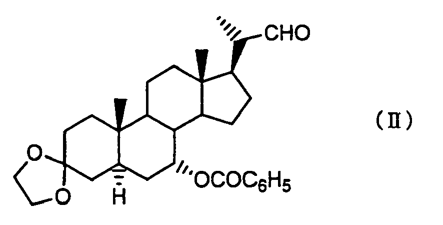 and a ketophosphonate metal salt of the formula (III) 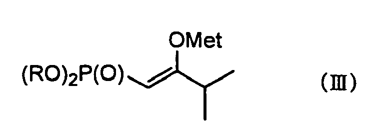 wherein R is an alkyl group or an aryl group, and Met is a metal atom.

3. The production method of claim 2, wherein the aldehyde compound is obtained by oxidizing a mixture of a solution containing an alcohol compound represented by the formula (IV) 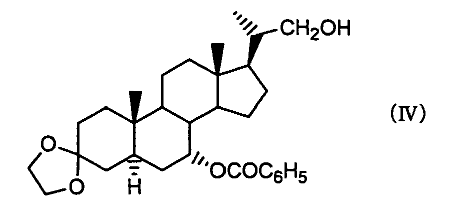 and a buffer with hypochlorite added as an oxidant, in the presence of an N-oxyl compound and within the pH range of an aqueous layer of 7 - 11.
